# EUROPEAN PATENT APPLICATION

(11) **EP 1 820 502 A1**
(43) Date of publication of application: **22.08.2007**
(21) Application number: 06384001.1
(22) Date of filing: 10.02.2006
(51) Int. Cl.: A61K 31/415, A61P 29/02

(54) **Active substance combination comprising azolylcarbinol compounds**

(71) Applicant: LABORATORIOS DEL DR. ESTEVE, S.A., 08041 Barcelona (ES)
(72) Inventor: Farre Gomis, Antonio, 08037 Barcelona (ES); Buschmann, Helmut Heinrich, 08960 Sant Just Desvern (BCN) (ES); Vela Hernandez, Jose Miguel, 08028 Barcelona (ES); Hamon, Michel, 75634 Paris, Cedex 13 (FR)
(74) Representative: Carpintero Lopez, Francisco

(57) **Abstract**

The present invention relates to active substance combination comprising at least one derivative of aryl (or heteroaryl) azolylcarbinol compounds and at least one gabapentinoid, a medicament comprising said active substance combination, a pharmaceutical formulation comprising said active substance combination and the use of said active substance combination for the manufacture of a medicament.

## Description

The present invention relates to active substance combination comprising at least one derivative of aryl (or heteroaryl) azolylcarbinol compounds and at least one gabapentinoid, a medicament comprising said active substance combination, a pharmaceutical formulation comprising said active substance combination and the use of said active substance combination for the manufacture of a medicament.

The treatment of pain conditions is of great importance in medicine. There is currently a world-wide need for additional pain therapy. The pressing requirement for a specific treatment of pain conditions or as well a treatment of specific pain conditions which is right for the patient, which is to be understood as the successful and satisfactory treatment of pain for the patients, is documented in the large number of scientific works which have recently and over the years appeared in the field of applied analgesics or on basic research on nociception.

Thus, it was an object of the present invention to provide a medicament that is suitable for the treatment of pain-related disorders.

Gabapentinoids are at present used for the treatment of pain, however, the therapeutical doses are relatively high.

Surprisingly it has been found that a combination of gabapentinoids and compounds of general formula (I) given above have a synergistic, clearly over-additive analgesic effect. Therefore, the dosage of at least one of the drugs of the combination used in pain-related disorders may be lowered and the incidence and intensity of undesired side effects may be reduced.

Thus, in one of its aspects the present invention relates to an active substance combination comprising
(A) at least one substituted azolylcarbinol compound of general formula I 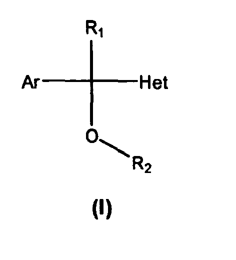 wherein
   Ar represents an optionally 1,2 or 3-fold substituted phenyl and/or thienyl radical, with substituents, equal or different, selected from the group consisting of fluorine, chlorine, bromine, methyl, trifluoromethyl and methoxy;
   R¹ represents a hydrogen atom or a lower alkyl group from C₁ to C₄;
   R² represents a dialkyl (C₁-C₄) aminoalkyl (C₂-C₃) radical; a (CH₂)-(CH₂)-NR³R⁴ group with R³ and R⁴, equal or different, representing hydrogen or a C₁₋₄-alkyl group; or azaheterocyclylalkyl (C₂-C₃); and
   Het represents an optionally 1 or 2-fold substituted azole, i.e. a five-membered nitrogenated aromatic heterocycle that contains one to three nitrogen atoms, with substituents, equal or different, selected from the group consisting of fluorine, chlorine, bromine and methyl;
   optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof,
   and/or
(B) at least one gabapentinoid and/or at least one of its analogues or derivatives optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

The term "lower alkyl group from C₁ to C₄", as referred to in the present invention, represents a linear or branched chain radical derived from a saturated hydrocarbon of 1 to 4 carbon atoms, such as methyl, ethyl, propyl, isopropyl, butyl, isobutyl, sec-butyl and tert-butyl.

The term "dialkyl (C₁-C₄)aminoalkyl (C₂-C₃), or azaheterocyclylalkyl (C₂-C₃)", as referred to in the present invention, represents an alkyl radical with two or three carbon atoms joined to a dialkyl (C₁-C₄) amine or to a cyclic amine, such as, for example, dimethylaminoethyl, dimethylaminopropyl, diethylaminoethyl, piperidinylethyl, morpholinylpropyl, pirrolidinylalkyl, etc.

Preferably, the active substance combination according to the present invention the combination comprises at least one compound of general formula (I) selected from the group consisting of
(+/-)-5-{alpha -[2-(dimethylamine)ethoxy]benzyl}-1-methyl-1H-pyrazole,
(+)-5-{alpha -[2-(dimethylamine)ethoxy]benzyl}-1-methyl-1H-pyrazole,
(-)-5-{alpha -[2-(dimethylamine)ethoxy]benzyl}-1-methyl-1H-pyrazole,
(-)-5-{alpha -[2-(dimethylamine)ethoxy]benzyl}-1-methyl-1H-pyrazole,
(+/-)-5-{alpha -[2-(dimethylamine)ethoxy]-2-thienylmethyl}-1-methyl-1H-pyrazole,
(+)-5-{alpha -[2-(dimethylamine)ethoxy]-2-thienylmethyl}-1-methyl-1H-pyrazole,
(-)-5-{alpha -[2-(dimethylamine)ethoxy]-2-thienylmethyl}-1-methyl-1H-pyrazole,
2-{α-[2-(dimethylamino)ethoxy]benzyl}-1-methyl-1*H*-imidazole,
N-methyl-2-((1-methyl-1H-pyrazol-5-yl)(phenyl)methoxy)ethanamine,
(S)-N-methyl-2-((1-methyl-1H-pyrazol-5-yl)(phenyl)methoxy)ethanamine,
(R)-N-methyl-2-((1-methyl-1H-pyrazol-5-yl)(phenyl)methoxy)ethanamine,
(±)-N,N-dimethyl-2-((1-methyl-1H-pyrazol-5-yl)(phenyl)methoxy)ethanamine,
(+)-N,N-dimethyl-2-((1-methyl-1H-pyrazol-5-yl)(phenyl)methoxy)ethanamine, and
(-)-N,N-dimethyl-2-((1-methyl-1H-pyrazol-5-yl)(phenyl)methoxy)ethanamine,
optionally in form of a corresponding salt thereof, or a corresponding solvate thereof.

The compounds of general formula (I) can be synthesized according to the procedures described in patents EP 289380 or WO 99/52525. These compounds of general formula (I) have a stereogenic center and the invention refers both to the use of a pure enantiomer and to the use of a mixture of enantiomers, The enantiomers can be prepared by any of the procedures described in our patents WO 97/20817, WO 99/02500, WO 99/07684 or WO 99/52525.

Gabapentinoids, such as gabapentin or pregabalin, are GABA analogues/derivatives and have been developed for treating epilepsy, neuropathic pain and bipolar disorders. GABA analogues/derivatives are compounds that are derived from or based on gamma-aminobutyric acid. GABA analogues/derivatives are either readily available or can be readily synthesized using known methods. Exemplary GABA analogues/derivatives and their salts preferably include gabapentin, pregabalin and their respective analogues/derivatives, and other GABA analogues/derivatives, as described in US 4,024,175, US 5,563,175, US 6,316,638, US 6,545,022, WO 93/23383, which are incorporated herein by reference.

In a preferred embodiment of the present invention the gabapentin analogues/derivatives are selected from the group consisting of

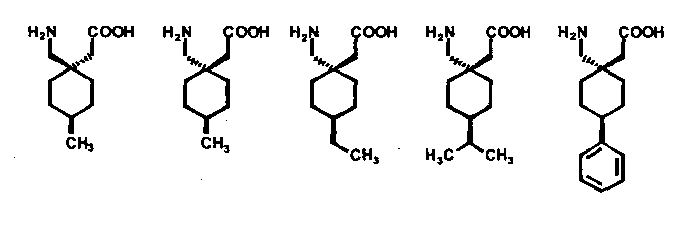

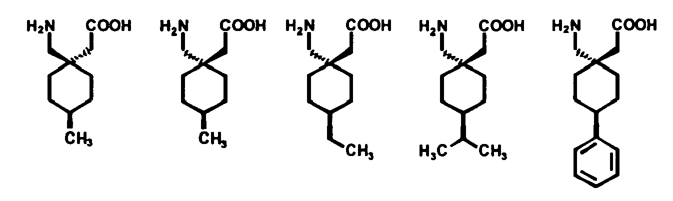

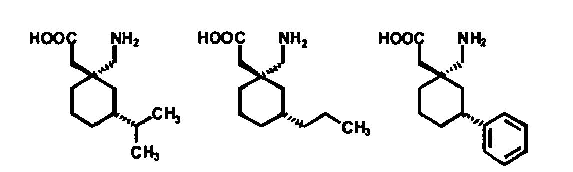

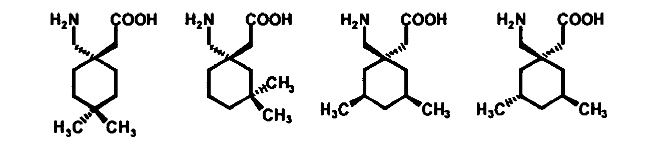

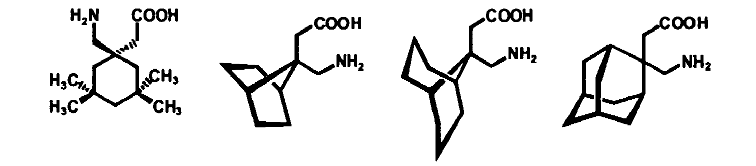

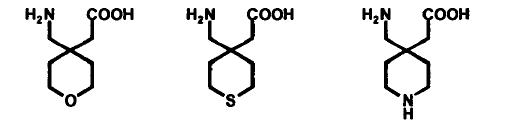

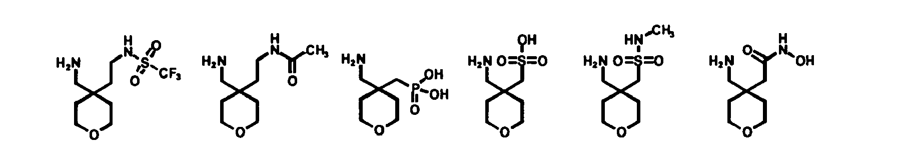

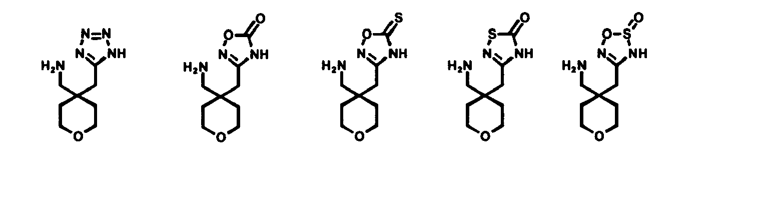

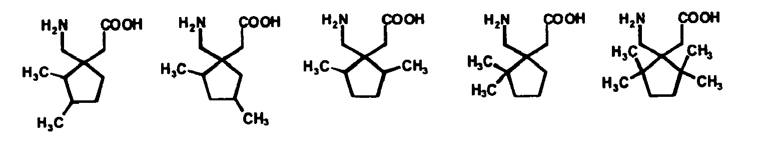

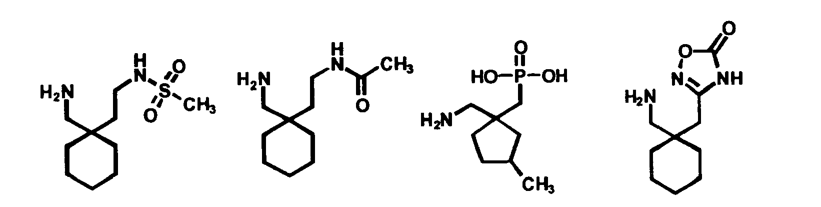

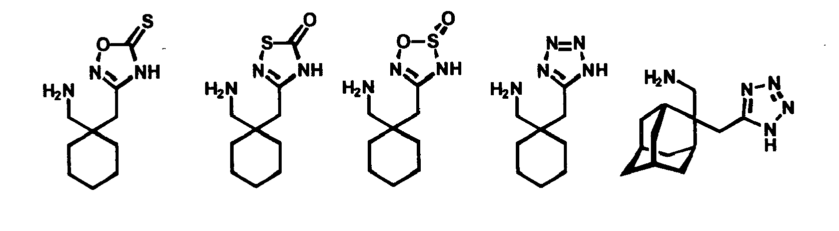

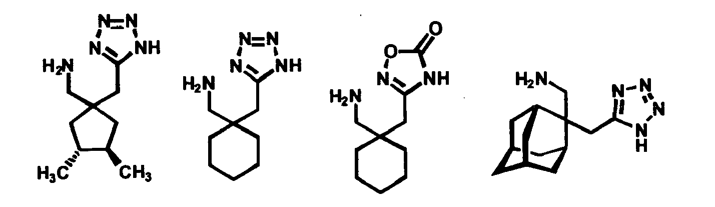

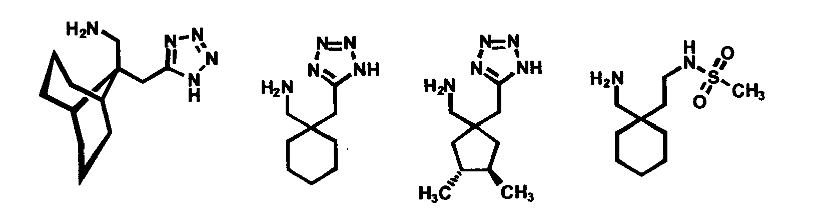

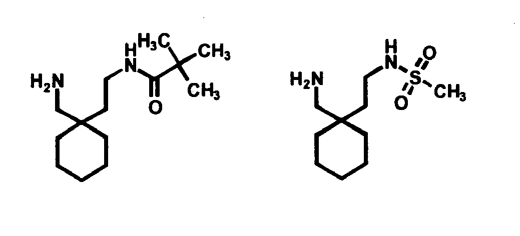

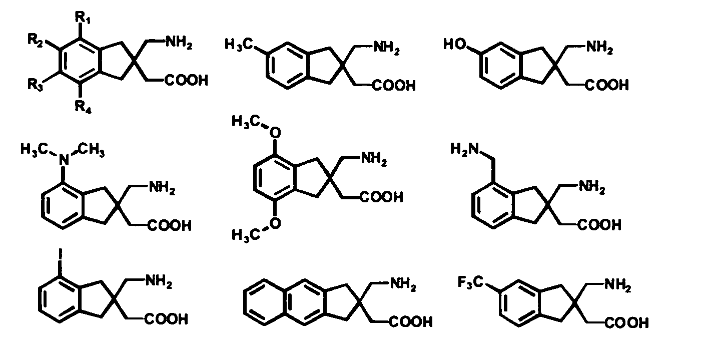

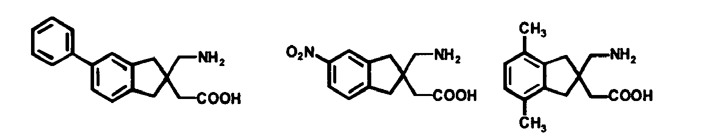

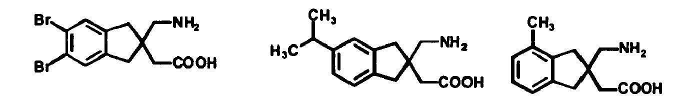

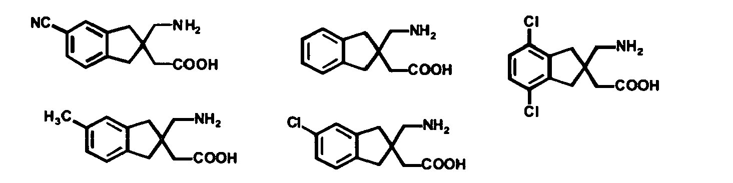

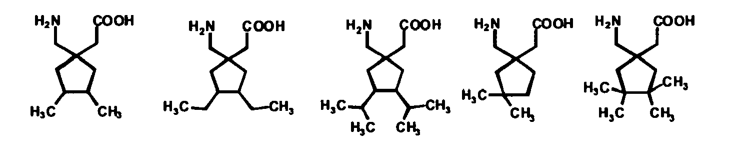

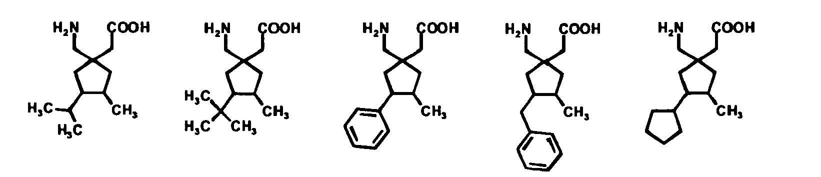

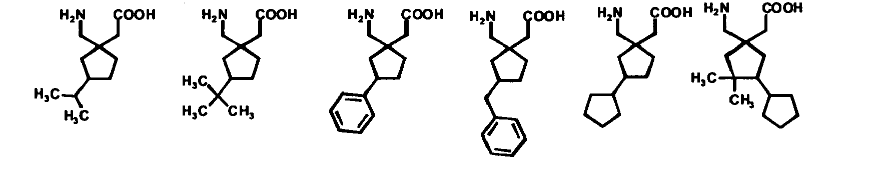

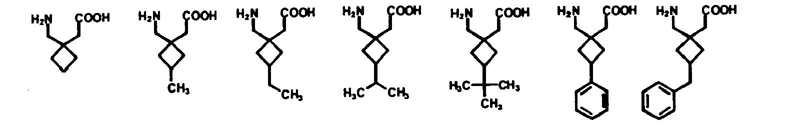

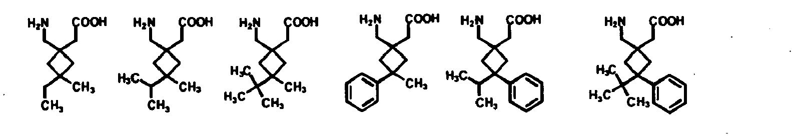

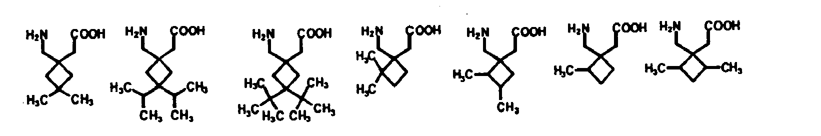

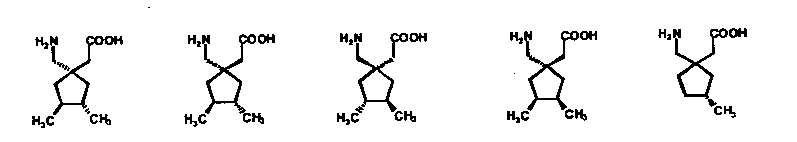

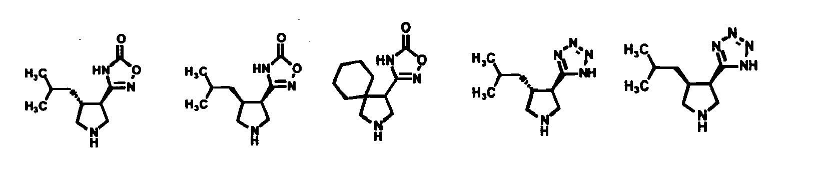

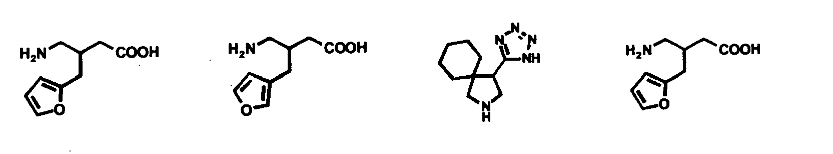

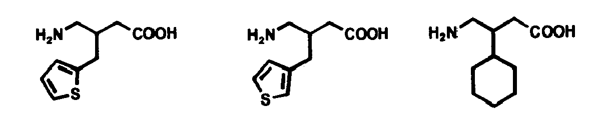

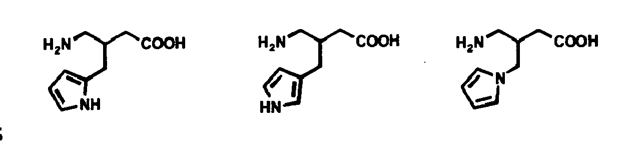

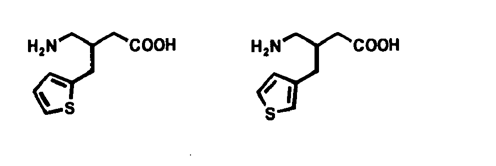

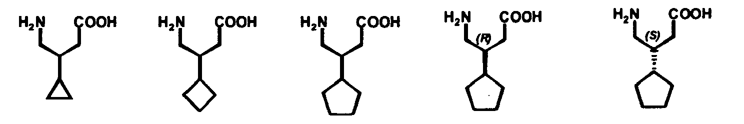

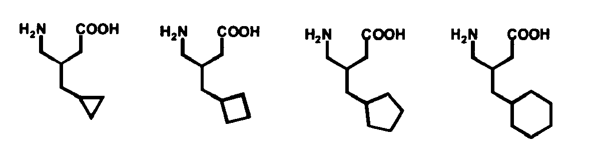

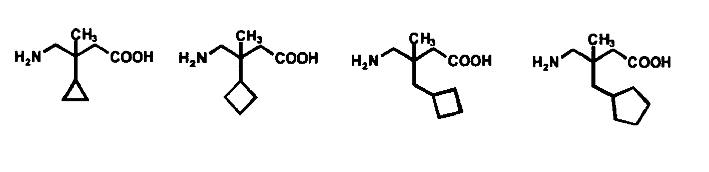

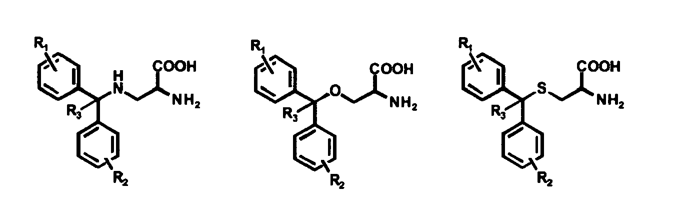

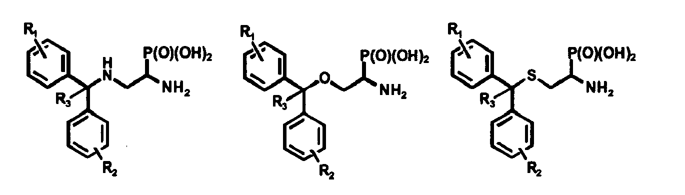

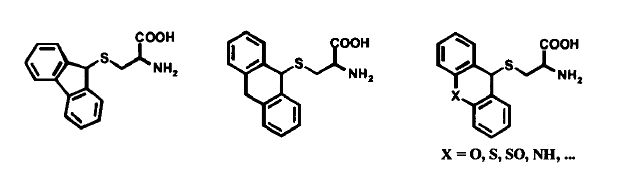

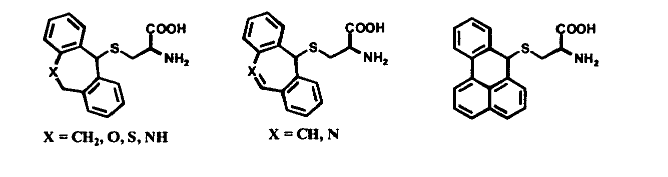

These above mentioned preferred gabapentin analogues/derivatives as well as their respective syntheses are described in J.S. Bryans et al., Biorg. Med. Chem. Lett. 1999, 9, 2329-2334; J.S. Bryans, D.J. Wustrow, Med. Res. Rev. 1999, 19, 149-177; J.S. Bryans et al., J. Org. Chem. 1998, 41, 1838-1845; WO99/21824, WO 99/31075, WO 2000/73259, WO 99/31057, WO 99/21824, WO 00/73300, WO 00/73296, WO 00/31020, US 6,166,072.

In connection with this invention "gabapentinoids" refer to compounds binding at the same site as gabapentin, or, alternatively, are acting like gabapentin or pregabalin. Preferred gabapentinoids are gamma-amino acids or analogues/derivatives thereof, GABA-mimetics, or GABA analogues/derivatives. Preferably, the gabapentinoids are gabapentin and pregablin.

The term "derivatives" as used in this application is defined here as meaning a chemical compound having undergone a chemical derivatization such as substitution or addition of a further chemical group to change (for pharmaceutical use) any of its physico-chemical properties, such as solubility or bioavailability. Derivatives include so-called prodrugs, e.g. ester and ether derivatives of an active compound that yield the active compound per se after administration to a subject. Examples of well known methods of producing a prodrug of a given acting compound are known to those skilled in the art and can be found e.g. in Krogsgaard-Larsen et al., "Textbook of Drugdesign and Discovery", Taylor & Francis (April 2002). The respective description is hereby incorporated by reference and forms part of the disclosure.

The term "analogues" as used in this application is defined here as meaning a chemical compound that is a derivative of a compound which has similar biochemical activity with respect to that compound.

Gabapentin (NEURONTIN^{®} or 1-(aminomethyl) cyclohexaneacetic acid) is an anticonvulsant drug with high binding affinity for certain calcium channel subunits. Although gabapentin was originally developed as a GABA-mimetic compound to treat spasticity, gabapentin has no direct GABAergic action and does not block GABA uptake or metabolism. (For review, see Rose et al. (2002) Anesthesia 57:451-462). However, gabapentin has been found to be an effective treatment for the prevention of partial seizures in patients who are refractory to other anticonvulsant agents. Gabapentin and the related drug pregabalin interact with [alpha]2[delta] subunits of calcium channels (Gee et al. (1996) J. Biol. Chem. 271: 5768-5776).

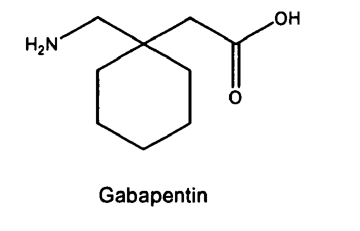

In addition to its known anticonvulsant effects, gabapentin has been shown to block the tonic phase of nociception induced by formalin and carrageenan, and exerts an inhibitory effect in neuropathic pain models of mechanical hyperalgesia and mechanical/thermal allodynia (Rose et al., Anaesthesia 57: 451-462 (2002)). Double-blind, placebo-controlled trials have indicated that gabapentin is an effective treatment for painful symptoms associated with diabetic peripheral neuropathy, post-herpetic neuralgia, and neuropathic pain (see, e.g., Mellegers et al., Clin. J. Pain 17:284-95 (2001)).

Pregabalin (LYRICA^{®}, (3S)-3-(aminomethyl)-5-methylhexanoic acid or (S)-(+)-3-isobutyl GABA; first described in: WO 92/09560) is another GABA analogue which shows anticonvulsant effects (Bryans et al., J. Med. Chem. 41:1838-1845 (1998)). Pregabalin has been demonstrated to possess even higher binding affinity for certain [alpha]2[delta] subunits of calcium channels than gabapentin (Bryans et al. Med. Res. Rev. 19:149-177 (1999)).

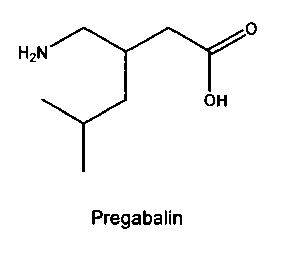

In a preferred embodiment of the present invention, component (B) is selected from the group consisting of 1-(Aminomethylcyclohexane acetic acid) optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the present invention, component (B) is selected from the group consisting of 3-(aminomethyl)-5-methylhexanoic acid, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the present invention compound (B) is Gabapentin or 1-(aminomethyl) cyclohexaneacetic acid, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the present invention compound (B) is Pregabalin, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

In another preferred embodiment of the present invention compound (B) is Pregabalin, (3S)-3-(aminomethyl)-5-methylhexanoic acid, (+)-4-Amino-3(S)-isobutylbutyric acid or (+)-(S)-3-Isobutyl-GABA optionally in form of a corresponding salt thereof, or a corresponding solvate thereof.

Yet, in another preferred embodiment of the present invention the active substance combination comprises component (A), selected from the group consisting of (±)-N,N-dimethyl-2-((1-methyl-1H-pyrazol-5-yl)(phenyl)methoxy)ethanamine, (+)-N,N-dimethyl-24(1-methyl-1H-pyrazol-5-yl)(phenyl)methoxy)ethanamine, N-methyl-2-((1-methyl-1H-pyrazol-5-yl)(phenyl)methoxy)ethanamine, (S)-N-methyl-2-((1-methyl-1H-pyrazol-5-yl)(phenyl)methoxy)ethanamine, (R)-N-methyl-2-((1-methyl-1H-pyrazol-5-yl)(phenyl)methoxy)ethanamine, and/or (-)-N,N-dimethyl-2-((1-methyl-1H-pyrazol-5-yl)(phenyl)methoxy)ethanamine, optionally in form of a corresponding salt thereof, or a corresponding solvate thereof, and/or component (B), comprising Gabapentin, optionally in form of a corresponding salt thereof, or a corresponding solvate thereof.

Yet, in another preferred embodiment of the present invention the active substance combination comprises component (A), selected from the group consisting of (±)-N,N-dimethyl-2-((1-methyl-1H-pyrazol-5-yl)(phenyl)methoxy)ethanamine, (+)-N,N-dimethyl-2-((1-methyl-1H-pyrazol-5-yl)(phenyl)methoxy)ethanamine, N-methyl-2-((1-methyl-1H-pyrazol-5-yl)(phenyl)methoxy)ethanamine, (S)-N-methyl-2-((1-methyl-1H-pyrazol-5-yl)(phenyl)methoxy)ethanamine, (R)-N-methyl-2-((1-methyl-1H-pyrazol-5-yl)(phenyl)methoxy)ethanamine, and/or (-)-N,N-dimethyl-2-((1-methyl-1H-pyrazol-5-yl)(phenyl)methoxy)ethanamine, optionally in form of a corresponding salt thereof, or a corresponding solvate thereof, and/or component (B), comprising Pregabalin, optionally in form of a corresponding salt thereof, or a corresponding solvate thereof.

Also, more preferably the active substance combination according to the present invention may as the azolylcarbinol compound of general formula I comprise one of the following compounds,

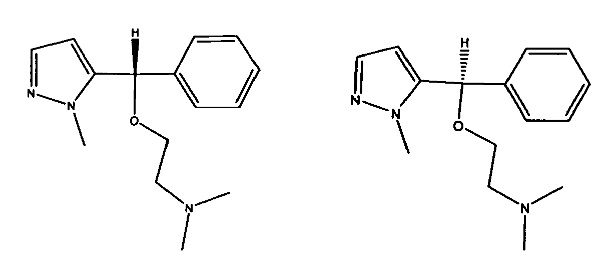

in each case optionally in the form of a corresponding N-oxide, a corresponding salt or a corresponding solvate thereof.

The inventive active substance combination may comprise the components (A) and (B) in a molar ratio of component (A) to component (B) in the range of 1:10 to 10:1, preferably 1:5 to 5:1.

The inventive active substance combination is suitable for the administration to humans, including infants, children and grown-ups, as well as animals.

Medicaments on the basis of the inventive active substance combination may preferably be administered once daily, twice daily or three times daily, more preferably once daily or twice daily, most preferably once daily.

In another aspect the present invention relates to a medicament comprising an inventive active substance combination and optionally at least one further active substance and/or optionally at least one auxiliary substance.

Preferably the inventive medicament is suitable for the treatment of pain.

"Treating" or "treatment" as used in this application are defined as including the treatment of the symptoms - of pain - as well as treatment of the disease or disease consequences causing the symptoms, the prevention or the prophylaxis of the symptoms - of pain - as well as the prevention or the prophylaxis of the disease or disease consequences causing the symptoms. Preferably "treating" or "treatment" as used in this application are defined as including the treatment of the symptoms - of pain - as well as treatment of the disease consequences causing the symptoms, the prevention or the prophylaxis of the symptoms - of pain - as well as the prevention or the prophylaxis of the disease consequences causing the symptoms. Most preferably "treating" or "treatment" as used in this application are defined as including the treatment of the symptoms of pain, and the prevention or the prophylaxis of the symptoms of pain.

PAIN is defined by the International Association for the Study of Pain (IASP) as "an unpleasant sensory and emotional experience associated with actual or potential tissue damage, or described in terms of such damage ( IASP, Classification of chronic pain, 2nd Edition, IASP Press (2002)). Even though pain is always subjective its causes or syndromes can be classified.

These well-defined pain syndromes include, according to the IASP central pain, peripheral pain, neuropathic pain, allodynia, causialgia, neuritis, hyperesthesia, neuralgia, hyperalgesia and hyperpathia. The characteristics of these pain syndromes can be found and are in detail described in: IASP, Classification of chronic pain, 2nd Edition, IASP Press (2002) p210, which is hereby included by reference.

Preferably the inventive medicament is suitable for the treatment of central pain, peripheral pain, peripheral neurogenic pain, central neuropathic pain, peripheral neuropathic pain, neuropathic pain, allodynia, causalgia, neuritis, hyperesthesia, neuralgia, hyperalgesia and/or hyperpathia.

More preferably, the inventive medicament is suitable for the treatment of neuropathic pain.

More preferably, the inventive medicament is suitable for the treatment of allodynia.

In a possibly preferred embodiment, the inventive medicament is preferably suitable for the treatment of acute inflammatory pain, acute pain, angina pain, bone injury pain, central pain, chronic daily headache, chronic lower back pain, chronic pain, cluster headaches, dental pain, fibromyalgia, fibromyalgia syndrome (FMS), genitourinary tract-related pain, herpes neuralgia, hyperalgesia, lancinating pain, migraine, myalgia, nociceptive pain, pain during labor and delivery, pain from cystitis, pain resulting from bums, pain resulting from sunburns, painful diabetic neuropathy, phantom limb pain, post partum pains, post-herpetic neuralgia, post-herpetic neuropathic pain, post-operative pain, refractory genitourinary pain, resistant pain, steady burning pain, surgical pain and/or visceral pain.

In a possibly preferred embodiment, the inventive medicament is preferably suitable for the treatment of chronic fatigue syndrome, neurogenic inflammation, diabetes, cystitis, gingivitis, dermatitis, psoriasis, inflammation of sciatic and lumbar nerves, gastrointestinal processes and/or ocular inflammation.

In another possibly preferred embodiment of the present invention, the inventive medicament is suitable for the treatment of diseases associated with excess of substance P selected from the group consisting of anxiety, depression, schizophrenia, manic depressive psychosis, sexual dysfunction, drug addiction, drug dependency, cognitive disorders and/or locomotive disorders.

In another possibly preferred embodiment of the present invention, the inventive medicament is suitable for the treatment of respiratory diseases selected from the group consisting of cough, bronchitis, chronic obstructive pulmonary diseases, allergic rhinitis and/or asthma.

In another possibly preferred embodiment of the present invention, the inventive medicament is suitable for the treatment of urinary incontinence disorders selected from the group consisting of urge incontinence, hyperreflexia; urinary stress incontinence, mixed incontinence and/or enuresis.

In another possibly preferred embodiment of the present invention, the inventive medicament is suitable for the treatment of diseases selected from the group consisting of epilepsy, seizures, motor neuron disease, osteoarthritis, muscle wasting disease, generalized anxiety disorder, social anxiety, panic disorders, and/or depression.

Another aspect of the present invention is the use of at least one active substance combination according to the present invention and optionally at least one further active substance and/or optionally at least one auxiliary substance for the manufacture of a medicament for the treatment of pain wherein the pain is preferably selected from the group consisting of neuropathic pain, allodynia, neuralgia, hyperesthesia, causalgia, neuritis, acute inflammatory pain, acute pain, angina pain, bone injury pain, central pain, chronic daily headache, chronic lower back pain, chronic pain, cluster headaches, dental pain, fibromyalgia, fibromyalgia syndrome (FMS), genitourinary tract-related pain, herpes neuralgia, hyperalgesia, lancinating pain, migraine, myalgia, nociceptive pain, pain during labor and delivery, pain from cystitis, pain resulting from bums, pain resulting from sunburns, painful diabetic neuropathy, phantom limb pain, post partum pains, post-herpetic neuralgia, post-herpetic neuropathic pain, post-operative pain, refractory genitourinary pain, resistant pain, steady burning pain, surgical pain and/or visceral pain.

Another possible embodiment of the present invention is the use of at least one active substance combination according to the present invention and optionally at least one further active substance and/or optionally at least one auxiliary substance for the manufacture of a medicament for the treatment of chronic fatigue syndrome, neurogenic inflammation, diabetes, cystitis, gingivitis, dermatitis, psoriasis, inflammation of sciatic and lumbar nerves, gastrointestinal processes and/or ocular inflammation.

Another possible embodiment of the present invention is the use of at least one active substance combination according to the present invention and optionally at least one further active substance and/or optionally at least one auxiliary substance for the manufacture of a medicament for the treatment of diseases associated with excess of substance P selected from the group consisting of anxiety, depression, schizophrenia, manic depressive psychosis, sexual dysfunction, drug addiction, drug dependency, cognitive disorders and/or locomotive disorders.

Another possible embodiment of the present invention is the use of at least one active substance combination according to the present invention and optionally at least one further active substance and/or optionally at least one auxiliary substance for the manufacture of a medicament for the treatment of respiratory diseases selected from the group consisting of cough, bronchitis, chronic obstructive pulmonary diseases, allergic rhinitis and/or asthma.

Another possible embodiment of the present invention is the use of at least one active substance combination according to the present invention and optionally at least one further active substance and/or optionally at least one auxiliary substance for the manufacture of a medicament for the treatment of urinary incontinence disorders selected from the group consisting of urge incontinence, hyperreflexia; urinary stress incontinence, mixed incontinence and/or enuresis.

Another possible embodiment of the present invention is the use of at least one active substance combination according to the present invention and optionally at least one further active substance and/or optionally at least one auxiliary substance for the manufacture of a medicament for the treatment of diseases selected from the group consisting of epilepsy, seizures, motor neuron disease, osteoarthritis, muscle wasting disease, generalized anxiety disorder, social anxiety, panic disorders, and/or depression.

A further aspect of the present invention relates to pharmaceutical formulations in different pharmaceutical forms comprising an inventive active substance combination and optionally at least one further active substance and/or optionally at least one auxiliary substance.

Preferably the inventive pharmaceutical formulation is suitable for oral or parenteral administration, more preferably for oral, intravenous, intraperitoneal, intramuscular, subcutaneous, intrathekal, rectal, transdermal, transmucosal or nasal administration.

Inventive pharmaceutical formulation for oral administration are preferably selected from the group consisting of tablets, drageés, capsules, powders, drops, gels, juices, sirups, solutions and suspensions.

The pharmaceutical formulation of the present invention for oral administration may also be in the form of multiparticulates, preferably microparticles, microtablets, pellets or granules, optionally compressed into a tablet, filled into a capsule or suspended in a suitable liquid. Suitable liquids are known to those skilled in the art.

The respective pharmaceutical formulations may - depending on their route of administration - also contain one or more auxiliary substances known to those skilled in the art. The pharmaceutical formulations according to the present invention may be produced according to standard procedures known to those skilled in the art, e.g. from the tables of contents from "Pharmaceutics: the Science of Dosage Forms", Second Edition, Aulton, M.E. (Ed.) Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 and "The Theory and Practice of Industrial Pharmacy", Lachman L., Lieberman H. and Kanig J. (Eds.), Lea & Febiger, Philadelphia (1986). The respective descriptions are incorporated by reference and are part of the present disclosure.

In one embodiment of the present invention the pharmaceutical formulation comprises one or both of the components (A) and (B) at least partially in a sustained-release form. Preferably the inventive pharmaceutical formulation comprises component (B) at least partially in a sustained-release form.

By incorporating one or both of these components at least partially or completely in a sustained-release form it is possible to extend the duration of their effect, allowing for the beneficial effects of such a sustained release form, e.g. the maintenance of even concentrations in the blood.

Suitable sustained-release forms as well as materials and methods for their preparation are known to those skilled in the art, e.g. from the tables of contents from "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000);"Controlled Drug Delivery", Vol. I, Basic Concepts, Bruck, S.D. (Ed.), CRC Press Inc., Boca Raton (1983) and from Takada, K. and Yoshikawa, H., "Oral Drug delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encylopedia of Controlled Drug Delivery, Mathiowitz, E. (Ed.), John Wiley & Sons, Inc., New York (1999), Vol. 2, 698-728. The respective descriptions are incorporated by reference and are part of the disclosure.

If the pharmaceutical formulation according to the present invention comprises at least one of the components (A) and (B) at least partially in a sustained-release form, said sustained release may preferably be achieved by the application of at least one coating or provision of a matrix comprising at least one sustained-release material.

The sustained-release material is preferably based on an optionally modified, water-insoluble, natural, semisynthetic or synthetic polymer, or a natural, semisynthetic or synthetic wax or fat or fatty alcohol or fatty acid, or on a mixture of at least two of these afore mentioned components.

The water-insoluble polymers used to produce a sustained-release material are preferably based on an acrylic resin, which is preferably selected from the group of poly(meth)acrylates, particularly preferably poly(C₁₋₄)alkyl (meth)acrylates, poly(C₁₋₄)dialkylamino(C₁₋₄)alkyl (meth)acrylates and/or copolymers or mixtures thereof, and very particularly preferably copolymers of ethyl acrylate and methyl methacrylate with a monomer molar ratio of 2:1 (Eudragit NE30D^{®}), copolymers of ethyl acrylate, methyl methacrylate and trimethylammonium ethyl methacrylate-chloride with a monomer molar ratio of 1:2:0.1 (Eudragit RS^{®}), copolymers of ethyl acrylate, methyl methacrylate and trimethylammonium ethyl methacrylate-chloride with a monomer molar ratio of 1:2:0.2 (Eudragit RL^{®}), or a mixture of at least two of the above-mentioned copolymers. These coating materials are commercially available as 30 wt.% aqueous latex dispersions, i.e. as Eudragit RS30D^{®}, Eudragit NE30D^{®} or Eudragit RL30D^{®}, and may also be used as such for coating purposes.

In another embodiment, the sustained-release material is based on water-insoluble cellulose derivatives, preferably alkyl celluloses, particularly preferably ethyl cellulose, or cellulose esters, e.g. cellulose acetate. Aqueous ethyl cellulose dispersions are commercially available, for example, under the trademarks Aquacoat^{®} or Surelease^{®}.

As natural, semisynthetic or synthetic waxes, fats or fatty alcohols, the sustained-release material may be based on carnauba wax, beeswax, glycerol monostearate, glycerol monobehenate, glycerol ditripalmitostearate, microcrystalline wax, cetyl alcohol, cetylstearyl alcohol or a mixture of at least two of these components.

The afore mentioned polymers of the sustained-release material may also comprise a conventional, physiologically acceptable plasticizer in amounts known to those skilled in the art.

Examples of suitable plasticizers are lipophilic diesters of a C₆-C₄₀ aliphatic or aromatic dicarboxylic acid and a C₁-C₈ aliphatic alcohol, e.g. dibutyl phthalate, diethyl phthalate, dibutyl sebacate or diethyl sebacate, hydrophilic or lipophilic citric acid esters, e.g. triethyl citrate, tributyl citrate, acetyltributyl citrate or acetyltriethyl citrate, polyethylene glycols, propylene glycol, glycerol esters, e.g. triacetin, Myvacet^{®} (acetylated mono- and diglycerides, C₂₃H₄₄O₅ to C₂₅H₄₇O₇), medium-chain triglycerides (Miglyol^{®}), oleic acid or mixtures of at least two of said plasticizers.

Aqueous dispersions of Eudragit RS^{®} and optionally Eudragit RL^{®} preferably contain triethyl citrate. The sustained-release material may comprise one or more plasticisers in amounts of, for example, 5 to 50 wt.% based on the amount of polymer(s) used.

The sustained-release material may also contain other conventional auxiliary substances known to those skilled in the art, e.g. lubricants, coloured pigments or surfactants.

The pharmaceutical formulation of the present invention may also comprise at least one of the components (A) and (B) covered by an enteric coating form which dissolves as a function of pH. Because of this coating, part or all of the pharmaceutical formulation can pass through the stomach undissolved and the components (A) and/or (B) are only released in the intestinal tract. The enteric coating preferably dissolves at a pH of between 5 and 7.5.

The enteric coating may be based on any enteric material known to those skilled in the art, e.g. on methacrylic acid/methyl methacrylate copolymers with a monomer molar ratio of 1:1 (Eudragit L^{®}), methacrylic acid/methyl methacrylate copolymers with a monomer molar ratio of 1:2 (Eudragit S^{®}), methacrylic acid/ethyl acrylate copolymers with a monomer molar ratio of 1:1 (Eudragit L30D-55^{®}), methacrylic acid/methyl acrylate/methyl methacrylate copolymers with a monomer molar ratio of 7:3:1 (Eudragit FS^{®}), shellac, hydroxypropyl methyl cellulose acetate-succinates, cellulose acetate-phthalates or a mixture of at least two of these components, which can optionally also be used in combination with the above-mentioned water-insoluble poly(meth)acrylates, preferably in combination with Eudragit NE30D^{®} and/or Eudragit RL^{®} and/or Eudragit RS^{®}.

The coatings of the pharmaceutical formulations of the present invention may be applied by the conventional processes known to those skilled in the art, e.g. from Johnson, J.L., "Pharmaceutical tablet coating", Coatings Technology Handbook (Second Edition), Satas, D. and Tracton, A.A. (Eds), Marcel Dekker, Inc. New York, (2001), 863-866; Carstensen, T., "Coating Tablets in Advanced Pharmaceutical Solids", Swarbrick, J. (Ed.), Marcel Dekker, Inc. New York (2001), 455-468; Leopold, C.S., "Coated dosage forms for colon-specific drug delivery", Pharmaceutical Science & Technology Today, 2(5), 197-204 (1999), Rhodes, C.T. and Porter, S.C., Coatings, in Encyclopedia of Controlled Drug Delivery. Mathiowitz, E. (Ed.), John Wiley & Sons, Inc., New York (1999), Vol. 1, 299-311. The respective descriptions are incorporated by reference and are part of the present disclosure.

In another embodiment, the pharmaceutical formulation of the present invention contains one or both of components (A) and (B) not only in sustained-release form, but also in non-retarded form. By combination with the immediately released form, a high initial dose can be achieved for the rapid onset of the beneficial effect. The slow release from the sustained release form then prevents the beneficial effect from diminishing. Such a pharmaceutical formulation is particularly useful for the treatment of acute health problems.

This may be achieved, for example, by a pharmaceutical formulation having at least one immediate-release coating comprising at least one of the components (A) and (B) to provide for rapid onset of the beneficial effect after administration to the patient.

The examples and figures in the following section describing pharmacological trials are merely illustrative and the invention cannot be considered in any way as being restricted to these applications.

### Example 1: Effects of Gabapentin + Cizolirtine on mechanical allodynia/hyperalgesia in rats with unilateral ligatures of the sciatic nerve

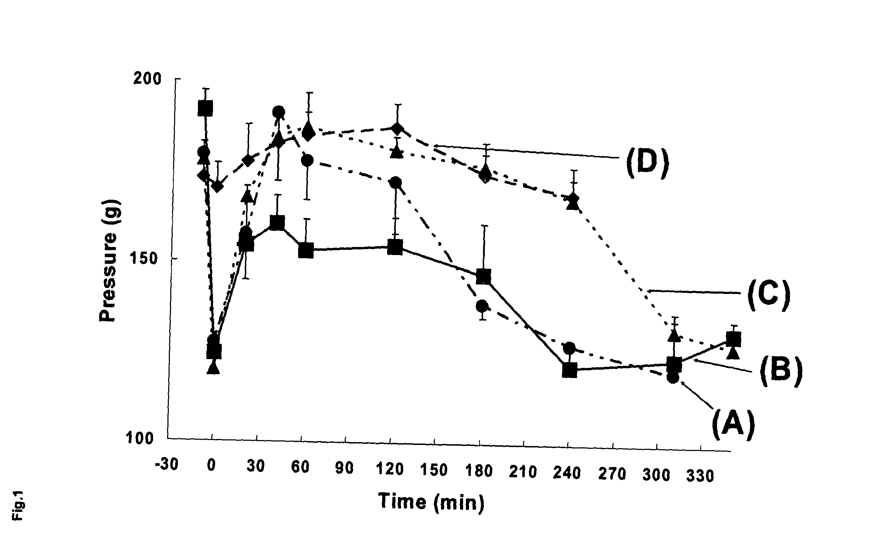

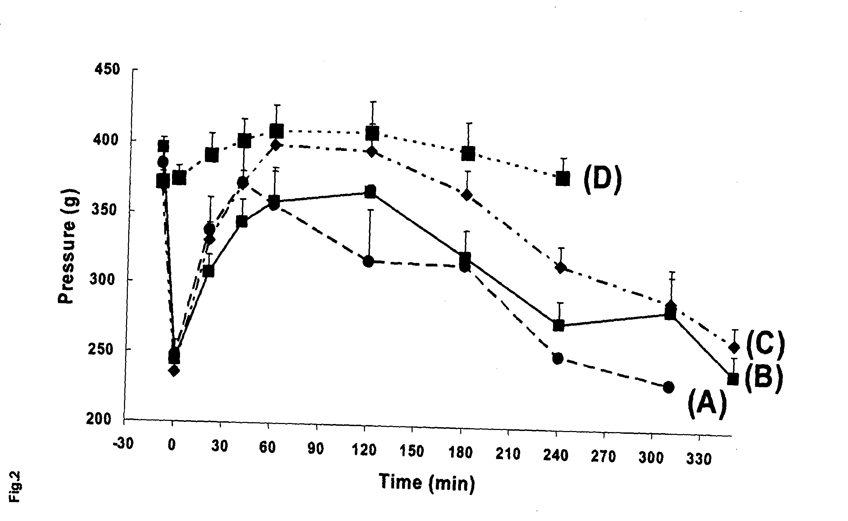

Ligatures of the right sciatic nerve were made in pentobarbital (50 mg/kg i.p.)-anesthetized rats according to the classical protocol of Bennett and Xie (1988) which is well known by those skilled in the art. Sham operated animals were subjected to the same surgical procedure except that the nerve was not ligated. Prior to surgery and two weeks afterwards, rats were subjected to the Randall-Selitto test to determine the pressure thresholds for triggering withdrawal of the ipsilateral, right hindpaw and vocalization. Animals were then immediately injected with either cizolirtine (5 mg/kg i.p.), gabapentin (30 mg/kg i.p.) or both drugs. In the latter case, a 5 min interval elapsed between the injection of cizolirtine (1st) and that of gabapentin (2^{nd}). Time 0 on the abscissa (see Figs 1 & 2) corresponded to the second injection. The Randall-Selitto test was then applied for the determination of pressure thresholds at various times up to at least 5 hours thereafter.

As shown in figure 1, unilateral ligatures of the sciatic nerve (0 on x-axis compared to the first, left hand side data points - corresponding to determinations made prior to ligatures) produced a marked decrease in pressure thresholds to trigger paw withdrawal with Cizolirtine ***(A)*** and Gabapentin ***(B)*,** respectively. However, concomitant administration of Cizolirtine and Gabapentin ***(C)*** produces the most pronounced analgesic effect comparable to sham-operated animals ***(D)*.** Moreover, these analgesic effects last significantly longer (at least 3 hours) when comparing with Cizolirtine and/or Gabapentin treatment only. The same results can be observed in vocalization experiments using the same experimental setup (figure 2). It is worth to mention that in all experimens performed, the combined administration of cizolirtine + gabapentin did not significantly increase pressure thresholds above control values (refering to intact non-operated rats), indicating that such treatment exerted no analgesic action.

Calculations of AUC (area under the curve) values from the curves (from 0 to 240-310 min on x-axis) depicted in figures 1 and 2 clearly showed that the effects of cizolirtine and gabapentin are at least additive (Figs 3 & 4).

### Figures

Figure 1: Randall-Selitto test to determine the pressure thresholds for triggering withdrawal of the ipsilateral, right, hindpaw. Both Cizolirtine and Gabapentin, respectively, decrease the pressure threshold to trigger paw withdrawal. However, concomitant administration of Cizolirtine and Gabapentin produces the most pronounced analgesic effect comparable to sham-operated animals. Moreover, these analgesic effects last significantly longer (at least 3 hours) when comparing with Cizolirtine and/or Gabapentin treatment only. *A: Cizolirtine; B: Gabapentin; C: Cizolirtine + Gabapentin; D: Sham-operated animals.*
Figure 2: Randall-Selitto test to determine the pressure thresholds for triggering vocalization. Both Cizolirtine and Gabapentin, respectively, decrease the threshold to trigger vocalization. However, concomitant administration of Cizolirtine and Gabapentin produces the most pronounced analgesic effect comparable to sham-operated animals. Moreover, these analgesic effects last significantly longer (at least 3 hours) when comparing with Cizolirtine and/or Gabapentin treatment only. *A: Cizolirtine; B: Gabapentin; C: Cizolirtine + Gabapentin; D: Sham-operated animals.*

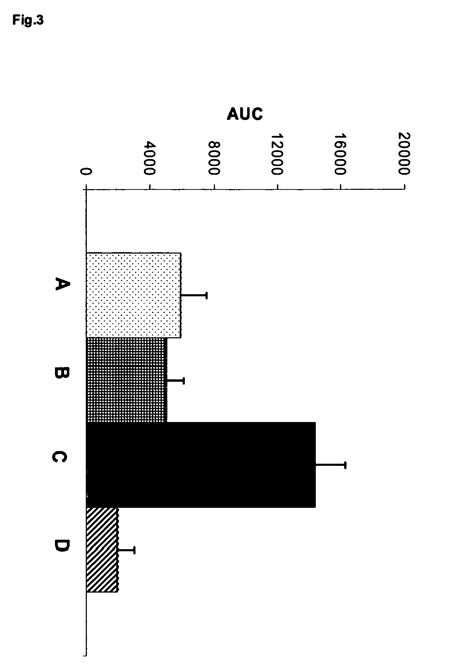

Figure 3: AUC calculations of paw withdrawal experiments shown in figure 1. *A: Cizolirtine; B: Gabapentin; C: Cizolirtine + Gabapentin; D: Sham-operated animals.*

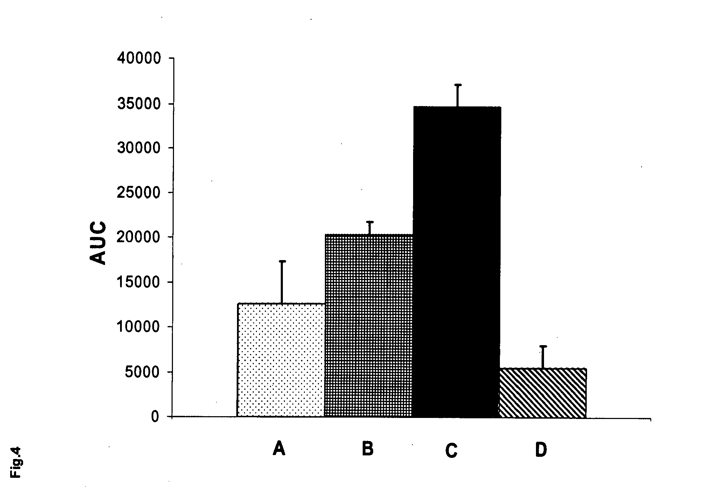

Figure 4: AUC calculations of vocalization experiments shown in figure 1. *A: Cizolirtine; B: Gabapentin; C: Cizolirtine + Gabapentin; D: Sham-operated animals.*

## Claims

1. Active substance combination comprising
A) at least one substituted azolylcarbinol compound of general formula I 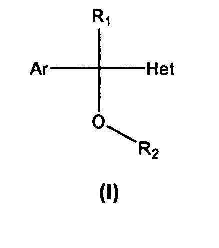 wherein
Ar represents an optionally 1,2 or 3-fold substituted phenyl and/or thienyl radical, with substituents, equal or different, selected from the group consisting of fluorine, chlorine, bromine, methyl, trifluoromethyl and methoxy;
R¹ represents a hydrogen atom or a lower alkyl group from C₁ to C₄;
R² represents a dialkyl (C₁-C₄) aminoalkyl (C₂-C₃) radical; a (CH₂)-(CH₂)-NR³R⁴ group with R³ and R⁴, equal or different, representing hydrogen or a C₁₋₄-alkyl group; or azaheterocyclylalkyl (C₂-C₃); and
Het represents an optionally 1 or 2-fold substituted azole, i.e. a five-membered nitrogenated aromatic heterocycle that contains one to three nitrogen atoms, with substituents, equal or different, selected from the group consisting of fluorine, chlorine, bromine and methyl;
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof,
and/or
(B) at least one gabapentinoid and/or at least one of its analogues or derivatives optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

2. Active substance combination according to claims 1, **characterized in that** component (B) is selected from the group consisting of 1-(Aminomethylcyclohexane acetic acid) optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

3. Active substance combination according to any of claims 1 to 2, **characterized in that** component (B) is selected from the group consisting of 3-(aminomethyl)-5-methylhexanoic acid optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

4. Active substance combination according to claim 1 or 2, **characterized in that** component (B) is Gabapentin or at least one of its analogues or derivatives, 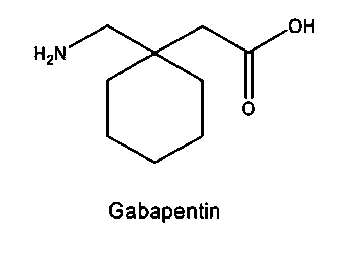 optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

5. Active substance combination according to claim 1 or 3, **characterized in that** component (B) is Pregabalin or at least one of its analogues or derivatives, 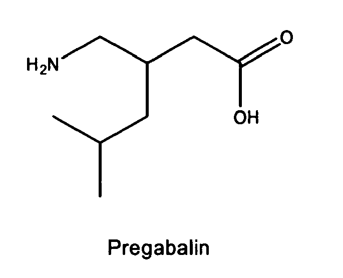 optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

6. Active substance combination according to any of claims 1-5, **characterized in that** component (A) is selected from the group consisting of
(+/-)-5-{alpha -[2-(dimethylamine)ethoxy]benzyl}-1-methyl-1H-pyrazole,
(+)-5-{alpha -[2-(dimethylamine)ethoxy]benzyl}-1-methyl-1H-pyrazoie,
(-)-5-{alpha -[2-(dimethylamine)ethoxy]benzyl}-1-methyl-1H-pyrazole,
(-)-5-{alpha -[2-(dimethylamine)ethoxy]benzyl}-1-methyl-1H-pyrazole,
(+/-)-5-{alpha -[2-(dimethylamine)ethoxy]-2-thienylmethyl}-1-methyl-1H-pyrazole,
(+)-5-{alpha -[2-(dimethylamine)ethoxy]-2-thienylmethyl}-1-methyl-1H-pyrazole,
(-)-5-{alpha -[2-(dimethylamine)ethoxy]-2-thienylmethyl}-1-methyl-1H-pyrazole,
N-methyl-2-((1-methyl-1H-pyrazol-5-yl)(phenyl)methoxy)ethanamine,
(S)-N-methyl-2-((1-methyl-1H-pyrazol-5-yl)(phenyl)methoxy)ethanamine,
(R)-N-methyl-2-((1-methyl-1H-pyrazol-5-yl)(phenyl)methoxy)ethanamine,
(±)-N,N-dimethyl-2-((1-methyl-1H-pyrazol-5-yl)(phenyl)methoxy)ethanamine,
(+)-N,N-dimethyl-2-((1-methyl-1H-pyrazol-5-yl)(phenyl)methoxy)ethanamine, and
(-)-N,N-dimethyl-2-((1-methyl-1H-pyrazol-5-yl)(phenyl)methoxy)ethanamine,
optionally in form of a corresponding salt thereof, or a corresponding solvate thereof.

7. Active substance combination according to any of claims 1, 2, 4 and 6, **characterized in that** the component (A) is selected from the group consisting of
(±)-N,N-dimethyl-2-((1-methyl-1H-pyrazol-5-yl)(phenyl)methoxy)ethanamine,
(+)-N,N-dimethyl-2-((1-methyl-1H-pyrazol-5-yl)(phenyl)methoxy)ethanamine,
N-methyl-2-((1-methyl-1H-pyrazol-5-ylXphenyl)methoxy)ethanamine,
(S)-N-methyl-2-((1-methyl-1H-pyrazol-5-yl)(phenyl)methoxy)ethanamine,
(R)-N-methyl-2-((1-methyl-1H-pyrazol-5-yl)(phenyl)methoxy)ethanamine,
and/or
(-)-N,N-dimethyl-2-((1-methyl-1H-pyrazol-5-yl)(phenyl)methoxy)ethanamine,
optionally in form of a corresponding salt thereof, or a corresponding solvate thereof,
and/or
component (B) is Gabapentin, optionally in form of a corresponding salt thereof, or a corresponding solvate thereof.

8. Active substance combination according to any of claims 1, 3, 5 and 6, **characterized in that** the component (A) is selected from the group consisting of
(±)-N,N-dimethyl-2-((1-methyl-1H-pyrazol-5-yl)(phenyl)methoxy)ethanamine,
(+)-N,N-dimethyl-2-((1-methyl-1H-pyrazol-5-yl)(phenyl)methoxy)ethanamine,
N-methyl-2-((1-methyl-1H-pyrazol-5-yl)(phenyl)methoxy)ethanamine,
(S)-N-methyl-2-((1-methyl-1H-pyrazol-5-ylXphenyl)methoxy)ethanamine,
(R)-N-methyl-2-((1-methyl-1H-pyrazol-5-yl)(phenyl)methoxy)ethanamine,
and/or
(-)-N,N-dimethyl-2-((1-methyl-1H-pyrazol-5-yl)(phenyl)methoxy)ethanamine,
optionally in form of a corresponding salt thereof, or a corresponding solvate thereof,
and/or
component (B) is Pregabalin, optionally in form of a corresponding salt thereof, or a corresponding solvate thereof.

9. Active substance combination according to one or more of claims 1, 2, 4, 6 and 7, **characterized in that** component (A) is one of the following compounds 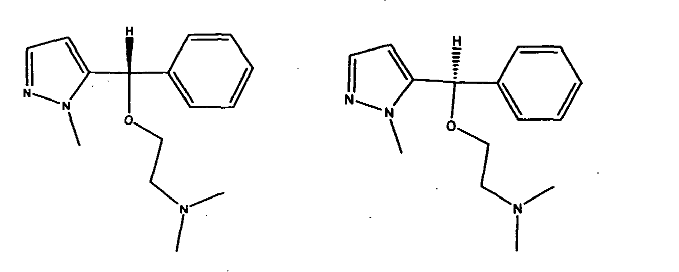 and/or
component (B) is Gabapentin, optionally in form of a corresponding salt thereof, or a corresponding solvate thereof.

10. Active substance combination according to one or more of claims 1, 3, 5, 6 and 8, **characterized in that** component (A) is one of the following compounds 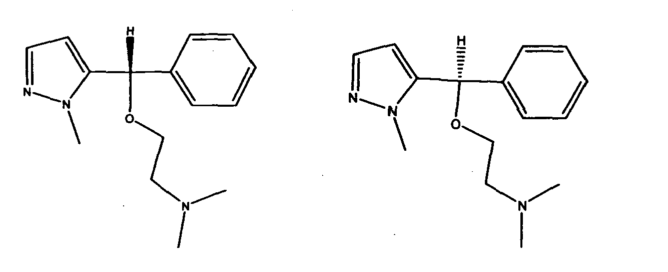 and/or
component (B) is Pregabalin, optionally in form of a corresponding salt thereof, or a corresponding solvate thereof.

11. Active substance combination according to one or more of claims 1-10, **characterized in that** the molar ratio of component (A) to component (B) is in the range of 1:10 to 10:1, preferably 1:5 to 5:1.

12. Medicament comprising an active substance combination according to one or more of claims 1 to 11 and optionally at least one further active substance and/or optionally at least one auxiliary substance.

13. Use of at least one active substance combination according to one or more of claims 1 to 12 and optionally at least one further active substance and/or optionally at least one auxiliary substance for the manufacture of a medicament for treatment of pain whereby said pain is preferably selected from the group consisting of neuropathic pain, allodynia, neuralgia, hyperesthesia, causalgia, neuritis, acute inflammatory pain, acute pain, angina pain, bone injury pain, central pain, chronic daily headache, chronic lower back pain, chronic pain, cluster headaches, dental pain, fibromyalgia, fibromyalgia syndrome (FMS), genitourinary tract-related pain, herpes neuralgia, hyperalgesia, lancinating pain, migraine, myalgia, nociceptive pain, pain during labor and delivery, pain from cystitis, pain resulting from bums, pain resulting from sunburns, painful diabetic neuropathy, phantom limb pain, post partum pains, post-herpetic neuralgia, post-herpetic neuropathic pain, post-operative pain, refractory genitourinary pain, resistant pain, steady burning pain, surgical pain and/or visceral pain; for the treatment of chronic fatigue syndrome, neurogenic inflammation, diabetes, cystitis, gingivitis, dermatitis, psoriasis, inflammation of sciatic and lumbar nerves, gastrointestinal processes and/or ocular inflammation; for the treatment of diseases associated with excess of substance P selected from the group consisting of anxiety, depression, schizophrenia, manic depressive psychosis, sexual dysfunction, drug addiction, drug dependency, cognitive disorders and/or locomotive disorders; for the treatment respiratory diseases selected from the group consisting of cough, bronchitis, chronic obstructive pulmonary diseases, allergic rhinitis and/or asthma; for the treatment of urinary incontinence disorders selected from the group consisting of urge incontinence, hyperreflexia; urinary stress incontinence, mixed incontinence and/or enuresis; for for the treatment of diseases selected from the group consisting of epilepsy, seizures, motor neuron disease, osteoarthritis, muscle wasting disease, generalized anxiety disorder, social anxiety, panic disorders, and/or depression.

14. Use according to claim 13 for the manufacture of a medicament for the treatment of pain whereby said pain is preferably selected from the group consisting of neuropathic pain, allodynia, neuralgia, hyperesthesia, causalgia, neuritis, acute inflammatory pain, acute pain, angina pain, bone injury pain, central pain, chronic daily headache, chronic lower back pain, chronic pain, cluster headaches, dental pain, fibromyalgia, fibromyalgia syndrome (FMS), genitourinary tract-related pain, herpes neuralgia, hyperalgesia, lancinating pain, migraine, myalgia, nociceptive pain, pain during labor and delivery, pain from cystitis, pain resulting from bums, pain resulting from sunburns, painful diabetic neuropathy, phantom limb pain, post partum pains, post-herpetic neuralgia, post-herpetic neuropathic pain, post-operative pain, refractory genitourinary pain, resistant pain, steady burning pain, surgical pain, and/or visceral pain.

15. Use according to claim 13 for the manufacture of a medicament for the treatment of chronic fatigue syndrome, neurogenic inflammation, diabetes, cystitis, gingivitis, dermatitis, psoriasis, inflammation of sciatic and lumbar nerves, gastrointestinal processes and/or ocular inflammation.

16. Use according to claim 13 for the manufacture of a medicament for the treatment of diseases associated with excess of substance P selected from the group consisting of anxiety, depression, schizophrenia, manic depressive psychosis, sexual dysfunction, drug addiction, drug dependency, cognitive disorders, and/or locomotive disorders.

17. Use according to claim 13 for the manufacture of a medicament for the treatment of respiratory diseases selected from the group consisting of cough, bronchitis, chronic obstructive pulmonary diseases, allergic rhinitis, and/or asthma.

18. Use according to claim 15 for the manufacture of a medicament for the treatment of urinary incontinence disorders selected from the group consisting of urge incontinence, hyperreflexia; urinary stress incontinence, mixed incontinence, and/or enuresis.

19. Use according to claim 13 for the manufacture of a medicament for the treatment of epilepsy, seizures, motor neuron disease, osteoarthritis, muscle wasting disease, generalized anxiety disorder, social anxiety, panic disorders, and/or depression.
